(19) Europäisches Patentamt
European Patent Office
Office européen des brevets

(11) Veröffentlichungsnummer: **0 068 281**
**B1**

# (12) EUROPÄISCHE PATENTSCHRIFT

(45) Veröffentlichungstag der Patentschrift: **05.12.84**

(21) Anmeldenummer: **82105225.5**

(22) Anmeldetag: **15.06.82**

(51) Int. Cl.³: **C 07 C 103/38**, C 07 C 103/737, C 07 C 103/78, C 07 D 307/12, C 08 G 18/66, C 08 G 18/14, C 08 K 5/20, C 08 J 9/38

(54) **Verwendung von Poly-N,N-hydroxyalkylamiden mehrwertiger Carbonsäuren als Zellöffner bei der Herstellung elastischer Polyurethanschaumstoffe.**

(30) Priorität: **25.06.81 DE 3124885**
**18.12.81 DE 3150269**

(43) Veröffentlichungstag der Anmeldung: **05.01.83 Patentblatt 83/1**

(45) Bekanntmachung des Hinweises auf die Patenterteilung: **05.12.84 Patentblatt 84/49**

(84) Benannte Vertragsstaaten: **BE DE FR GB IT NL SE**

(56) Entgegenhaltungen:
**EP - A - 0 028 469**
**AT - B - 294 429**
**DE - A - 1 745 134**
**DE - A - 2 509 237**
**GB - A - 2 010 265**
**GB - A - 2 025 439**

**Chemical Abstracts Band 95, Nr. 8 24. August 1981 Columbus, Ohio, USA G.A. YANGOL et al. "Poly(ether-amide-urethane) coatings" Seite 81, Spalte 1, Abstract No. 63778s**
**CHEMICAL ABSTRACTS Band 81, Nr. 7 19. August 1974 Columbus, Ohio, USA T. KOBAYASHI et al. "Amido acrylates" Seite 363, Spalte 2, Abstract Nr. 37268s**

(73) Patentinhaber: **BAYER AG, Konzernverwaltung RP Patentabteilung, D-5090 Leverkusen 1 Bayerwerk (DE)**

(72) Erfinder: **Haas, Peter, Dr., Zwengenberger Strasse 43, D-5657 Haan 1 (DE)**
Erfinder: **Hettel, Hans, Dr., Hahnenweg 5, D-5000 Koeln 80 (DE)**

**EP 0 068 281 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung ist die Verwendung von Poly-N,N-hydroxyalkylamiden mehrwertiger Carbonsäuren als Zellöffner bei der Herstellung hochelastischer Polyurethanschaumstoffe.

Die Erfahrung bei der technischen Herstellung hochelastischer Polyurethanschaumstoffe zeigt, dass die Produktion trotz eines hohen Standes der technischen Entwicklung immer noch mit Risiken bezüglich der Schaumstoffqualität verbunden ist. An erster Stelle ist hierbei die Schrumpfanfälligkeit zu benennen, die zu einer uneinheitlichen Verteilung der mechanischen Eigenschaften über den Blockquerschnitt infolge einer Randzonenverdichtung führt. Besonders stark ist hier der gesamte Oberflächenbereich betroffen, der dann als Ausschuss für die Verwertung verlorengeht.

Aufgabe der Erfindung ist daher, ein Verfahren zur Herstellung hochelastischer und über den gesamten Blockquerschnitt offenzelliger elastischer Polyurethanschaumstoffe mit drastisch reduzierter Tendenz zu Schrumpferscheinungen und erleichterter Aufdrückbarkeit zur Verfügung zu stellen, das durch die Mitverwendung von Poly-N,N-hydroxyalkylamiden mehrwertiger Carbonsäuren in der schaumstoffbildenden Reaktion gekennzeichnet ist. In den so hergestellten Schaumstoffen wird gleichzeitig ein hohes Mass an Formstabilität bei der Weiterverarbeitung gewährleistet.

Es war ein überraschendes Moment der Erfindung, dass geringe Mengen an Poly-N,N-hydroxyalkylamiden mehrwertiger aliphatischer, cycloaliphatischer und/oder aromatischer Carbonsäuren in Polyurethanschaumstoffrezepturen ein so hohes Mass an Offenzelligkeit bewirken und dabei gleichzeitig die Schrumpftendenz erheblich reduzieren.

Überraschend ist weiterhin der Befund, dass bei der erfindungsgemässen Verwendung der in den Patentansprüchen definierten Zellöffner es möglich geworden ist, modifizierte (verzweigte) aromatische Diisocyanattypen, z.B. Toluylendiisocyanattypen, mit beispielsweise Allophanat-, Biuret-, Isocyanurat-, Carbodiimid-, Urethan- und/oder Harnstoffgruppen auch in Gegenwart von metallorganischen Urethanbildungskatalysatoren, speziell Organozinnverbindungen wie Zinn(II)carboxylaten oder Dialkylzinn(IV)dicarboxylaten zu verschäumen, ohne dass es hierbei, wie sonst üblich, in den Schaumstoffen zu starken Schrumpfeffekten kommt. Auch in diesen Fällen der Zinnkatalyse wird ein stark offenzelliger, schrumpffreier Schaumstoff erhalten, der durch die zusätzlich möglich gewordene Metallkatalyse darüber hinaus ein verbessertes mechanisches Werteniveau, z.B. ersichtlich an der Stauchhärte, zeigt.

Die gleichen Probleme bestanden bisher auch bei den stark funktionalisierten (verzweigten), polymeren Diphenylmethanpolyisocyanaten bei ihrer Verwendung in der Schaumstoffherstellung. Auch hier wirken die erfindungsgemäss beanspruchten Verbindungen eindeutig als Zellöffner.

Bei der Herstellung von Kaltschaumstoffen war es bislang üblich, den Schrumpf- und Zellstrukturproblemen durch Stabilisatoren (z.B. wie in US-PS Nr. 3748288, DE-A Nrn. 2210721 und 2454049) teilweise entgegenzuwirken, musste jedoch bekanntlich eine Senkung der mechanischen Eigenschaften, insbesondere der Stauchhärte, hinnehmen, was gleichzeitig die Grenze dieser Verfahren aufzeigte. So beschreibt die DE-A Nr. 2103730 zwar die Herstellung von offenporigen Schaumstoffen durch Verwendung von Polyalkylenpolyaminen als einbaufähige Katalysatoren, jedoch muss hierbei, wie in der Offenlegungsschrift auf S. 6, Abs. 2, beschrieben, auf die Organozinnkatalyse wegen der hierdurch bewirkten Schrumpfeffekte verzichtet werden. Durch die erfindungsgemässe Verfahrensweise unter Verwendung der Polyhydroxyalkylamide der mehrwertigen Carbonsäuren als Zellöffner kann das mechanische Werteniveau nicht nur gehalten, sondern durch die jetzt auch mögliche Metallkatalyse sogar noch gesteigert werden, wobei gleichzeitig eine hohe Offenzelligkeit und eine stark reduzierte Schrumpfneigung erreicht wird. Die zellöffnende Wirkung der erfindungsgemäss verwendeten Verbindungen kann sowohl in Block- als auch in Formschaumstoffen signifikant aufgezeigt werden.

Die Effekte, die für eine erhebliche Steigerung der Verarbeitungssicherheit bei der Schaumstoffherstellung von Bedeutung sind, können nicht mit einer Vernetzungsfunktion der erfindungsgemäss verwendeten Stoffklasse erklärt werden, wie dies eigentlich erwartet werden sollte. Bei einer solchen Vernetzerwirkung der erfindungsgemäss einzusetzenden Stoffklasse würden nämlich bei hoher Dosierung erfahrungsgemäss infolge Übervernetzung starke Schrumpferscheinungen bewirkt werden. Das Gegenteil ist jedoch der Fall; bei zu hoher Konzentration verursacht die erfindungsgemäss verwendete Verbindungsklasse einen Schaumstoffkollaps als Folge der gesteigerten Zellöffnerwirkung.

Aus der GB-PS Nr. 1032873 sind bereits Poly-N,N-hydroxyalkylamide von Polymerfettsäuren der Formel

$$R-\underset{\underset{O}{\|}}{C}.N(C_1\text{-}C_8\text{-aliph. Reste}-OH)_{2\text{-}4}$$

bekannt, wobei R der di-, tri- oder polymerisierte Rest von Fettsäuren mit 8 bis 24 C-Atomen ist. Diese Verbindungen wurden mit Polyisocyanaten zur Herstellung von Beschichtungen verwendet.

Die erfindungsgemäss verwendeten Verbindungen besitzen jedoch eine andere Zusammensetzung und werden für einen völlig anderen Zweck, nämlich als Zusatzstoffe bei der Polyurethanschaumstoffherstellung mit der Wirkung als Zellöffner verwendet.

In „Chem. Abstract", *95* (1981), „Abstract" Nr. 63778 s ist die Verwendung von Poly-N,N-hydroxyalkylamiden zur Umsetzung mit Polyisocyanaten und weiteren, reaktionsfähige Wasserstoffatome aufweisenden Verbindung zur Herstellung von Poly(etheramidurethan)lacken bzw.

-überzügen beschrieben. In solchen Lacken führt der teilweise oder vollständige Ersatz des trifunktionellen Vernetzers Trimethylolpropan durch die Poly-N,N-hydroxyalkylamide zu erhöhtem *potlife* und erhöhter relativer Härte unter Beibehaltung hoher Zugfestigkeit, aber zumeist verminderter Hitzebeständigkeit der Überzüge.

In der DE-A Nr. 1745134 werden laut Beispielen Poly-N,N-hydroxyalkylamide zweibasischer aromatischer Carbonsäuren als alleinige Polyole oder in überwiegenden Mengen von 60 bis 90 Gew.-Teilen neben 10 bis 40 Gew.-Teilen üblicher, nieder- oder höhermolekularer Polyole zur Herstellung von flammwidrigen Polyurethan-(hart)schaumstoffen eingesetzt, wobei organische Treibmittel laut Beispielen zur Bildung der Hartschaumstoff dienen. Die entstehenden Hartschaumstoffe bestehen dabei überwiegend aus geschlossenen Zellen (s. Beispiele 1 bis 17).

Erfindungsgemäss sind als Zellöffner in geringen Einsatzmengen Poly-N,N-hydroxyalkylamide mehrwertiger aliphatischer, cycloaliphatischer und/oder aromatischer Polycarbonsäuren der Formel

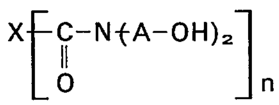

zu verwenden, wobei in der Formel n eine ganze Zahl von 2 bis 6, bevorzugt von 2 bis 4, X nur eine Bindung (bei n = 2), ein n-wertiger $C_1$ bis $C_{10}$-geradkettiger oder verzweigter, gegebenenfalls hydroxylgruppensubstituierter Alkanrest, vorzugsweise ein $C_1$ bis $C_4$-Alkanrest, ein $C_4$ bis $C_6$-Cycloalkanrest, der im Ring gegebenenfalls O, S oder $N-CH_3$ als Heteroatom oder Heteroatomgruppe enthält, ein n-wertiger, $C_6$ bis $C_{20}$-Arylrest, der gegebenenfalls durch eine oder mehrere Hydroxylgruppen substituiert ist, A eine geradkettige oder verzweigte, gegebenenfalls eine OH-Gruppe aufweisende $C_2$ bis $C_6$-Alkylengruppe, vorzugsweise eine Ethylengruppe, eine 1,2- oder 1,3-Propangruppe, besonders bevorzugt eine Ethylengruppe, darstellt.

Bevorzugt sind Verbindungen, wo
X nur eine Bindung (n = 2)
ein $C_1$ bis $C_6$-Alkylenrest (n = 2)

ein HC $\lessdot$-Rest (n = 3)
ein $H_3C.C$ $\lessdot$-Rest (n = 3)
ein HO−C $\lessdot$-Rest (n = 3)

ein 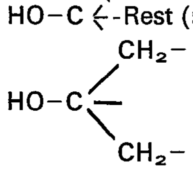 -Rest (n = 3)

ein 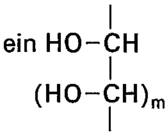 -Rest (n = 2)

(m = 1-3)

ein 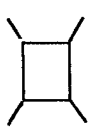 -Rest (n = 4)

ein 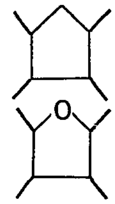 -Rest (n = 4) und

ein -Rest (n = 4)

ein 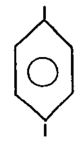 -Rest (n = 2)

ein 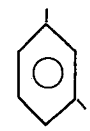 -Rest (n = 2)

ein 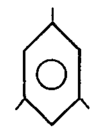 -Rest (n = 3)

ein 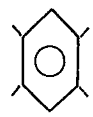 -Rest (n = 4)

ein 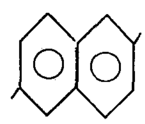 -Rest (n = 2)

ein 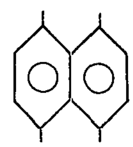 -Rest (n = 4)

ein 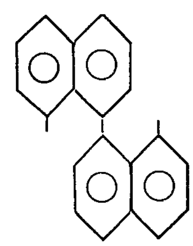 -Rest (n = 2)

ein 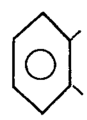 -Rest (n = 2)

ein 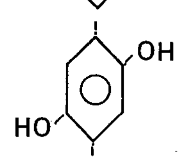 -Rest (n = 2)

oder

ein 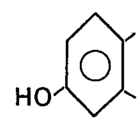 -Rest (n = 2) ist

Besonders bevorzugt sind Di(hydroxyalkyl)-amide für X = nur eine Bindung,

= 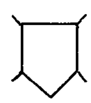

= $-(CH_2)_m-$ mit m gleich 1 bis 4

= 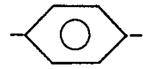

= 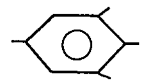

= 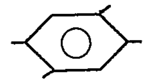

= 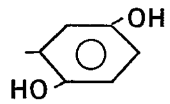

Die Verfahren zur Herstellung der erfindungsgemäss zu verwendeten Poly-N,N-hydroxyalkylamide mehrwertiger Polycarbonsäuren sind im Prinzip bekannt. Die Verbindungen können demgemäss durch Umsetzung von Polycarbonsäurealkyl- oder -arylestern der Formel

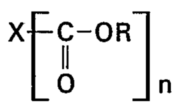

wobei X und n die angeführte Bedeutung haben und R einen $C_1$ bis $C_{10}$- (vorzugsweise $C_1$ bis $C_4$-) Alkylrest oder einen Arylrest (vorzugsweise Phenylrest) darstellt, mit Dialkanolaminen der Formel

$$HN(A-OH)_2$$

wobei A die angeführte Bedeutung hat, bei erhöhten Temperaturen (50 bis 200, vorzugsweise 70 bis 150°C) und Abdestillieren der abgespaltenen Hydroxylverbindungen ROH aus dem Reaktionsgemisch bei Normaldruck und/oder vermindertem Druck erhalten werden. Entsprechende Aminolyseverfahren sind z.B. in Houben-Weyl, Bd. 8, S. 653 ff. und Bd. XI/2, S. 27 sowie in der GB-PS Nr. 1032873 beschrieben.

Gegenstand der Erfindung ist somit die Verwendung von Poly-N,N-hydroxyalkylamiden mehrwertiger Polycarbonsäuren bei der Herstellung von Polyurethanschaumstoffen, dadurch gekennzeichnet, dass man für die Herstellung von offenzelligen, elastischen Polyurethanschaumstoffen durch Umsetzung von mindestens 2 gegenüber Isocyanaten reaktionsfähige Wasserstoffatome aufweisenden Verbindungen vom Molekulargewicht 400 bis 10 000 mit Polyisocyanaten und gegebenenfalls Kettenverlängerungsmitteln vom Molekulargewicht 18 bis 400, in Gegenwart von Katalysatoren und Wasser als Treibmittel und gegebenenfalls an sich bekannten Hilfs- und Zusatzmitteln, Poly-N,N-hydroxyalkylamide mehrwertiger aliphatischer, cycloaliphatischer und/oder aromatischer Polycarbonsäuren der Formel

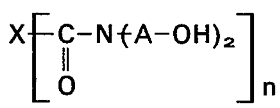

wobei n eine ganze Zahl von 2 bis 6, X nur eine Bindung (bei n = 2), ein n-wertiger $C_1$ bis $C_{10}$-geradkettiger oder verzweigter, gegebenenfalls hydroxylgruppensubstituierter Alkanrest, eine $C_4$ bis $C_6$-Cycloalkanrest, der im Ring gegebenenfalls O, S oder $N-CH_3$ als Heteroatom oder Heteroatomgruppe enthält, ein n-wertiges $C_6$ bis $C_{10}$-Arylrest, der gegebenenfalls durch eine oder mehrere Hydroxylgruppen substituiert ist, und A eine geradkettige oder verzweigte, gegebenenfalls eine OH-Gruppe aufweisende $C_2$ bis $C_6$-Alkylengruppe, darstellt,
in Mengen von 0,1 bis 7,5, zumeist 0,5 bis 7,5 und bevorzugt 0,75 bis 5 Gew.-%, bezogen auf das Gemisch mit den mindestens 2 gegenüber Isocyanaten reaktionsfähige Wasserstoffe aufweisenden Verbindungen vom Molekulargewicht 400 bis 10 000, einsetzt.

Bei den Verbindungen mit zellöffnender Wirkung handelt es sich um am Amidstickstoff perhydroxyalkylierte Derivate von Amiden mehrwertiger Carbonsäuren, wie z.B.

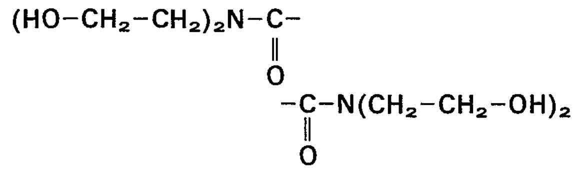

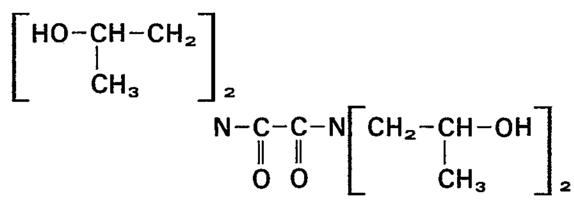

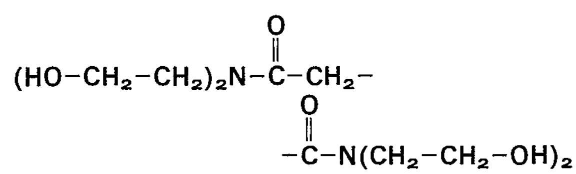

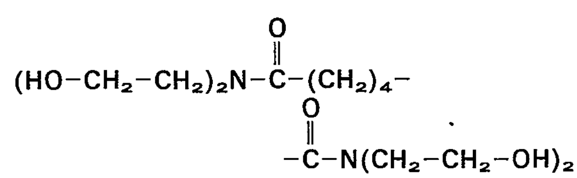

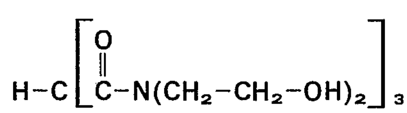

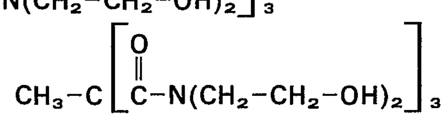

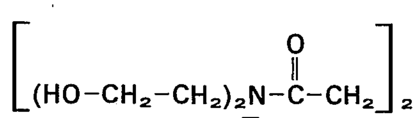

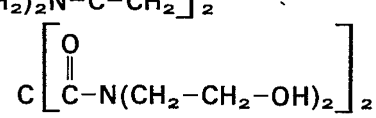

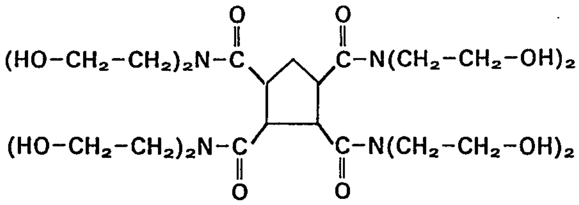

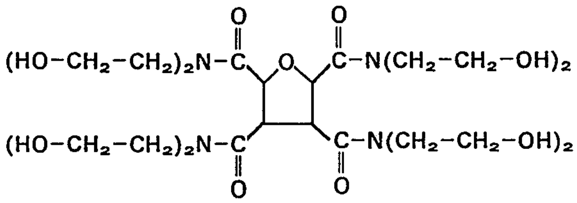

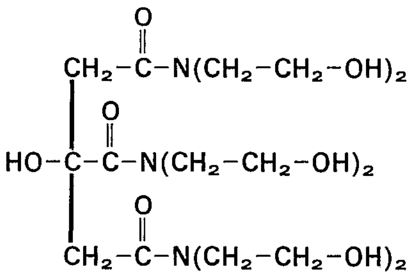

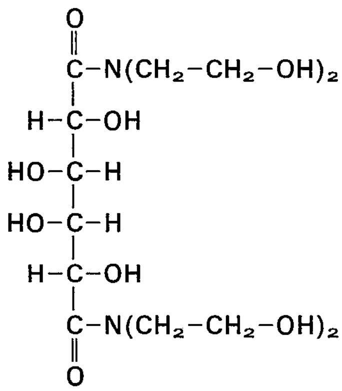

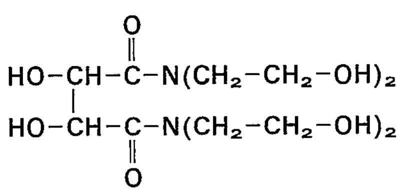

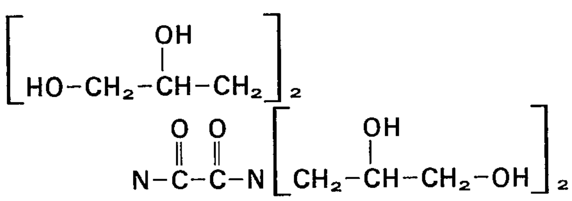

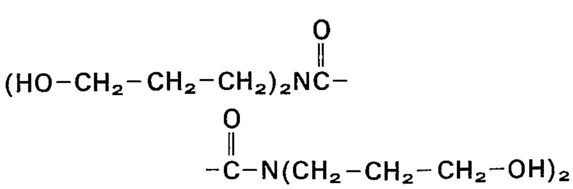

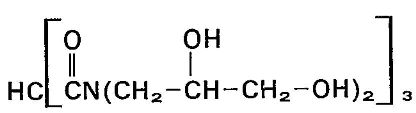

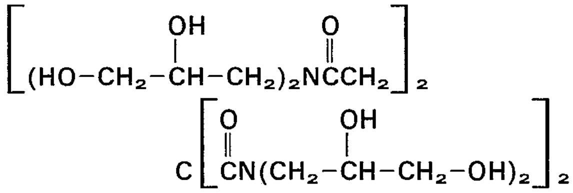

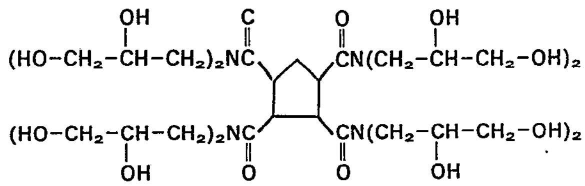

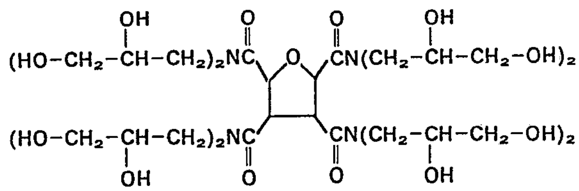

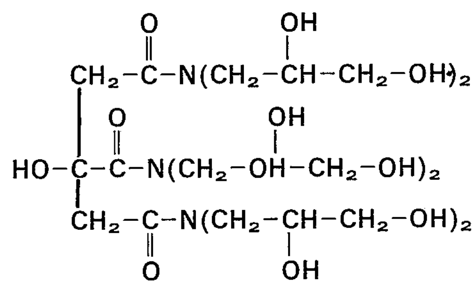

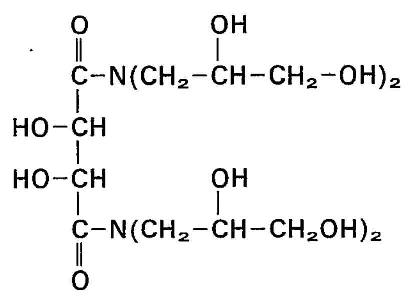

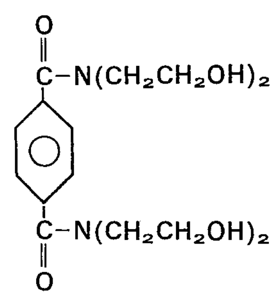

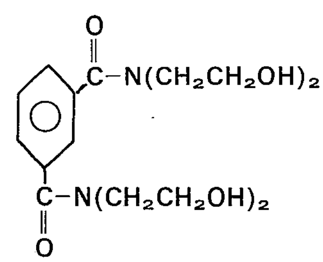

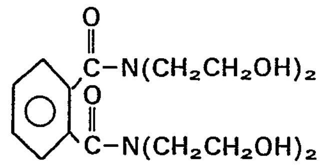

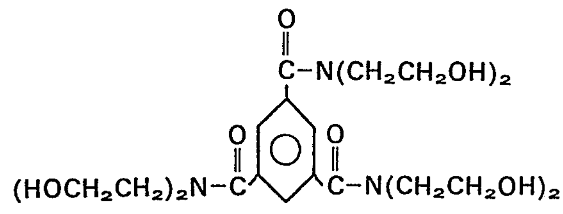

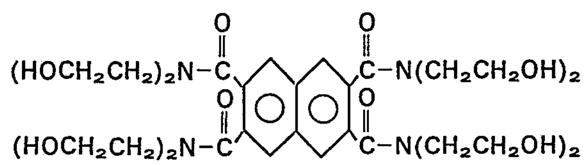

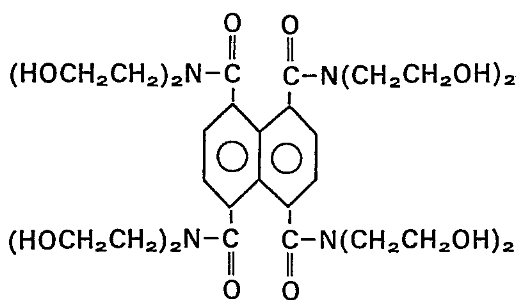

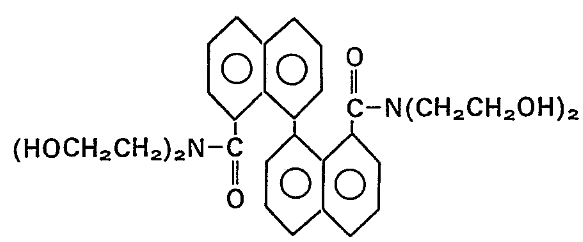

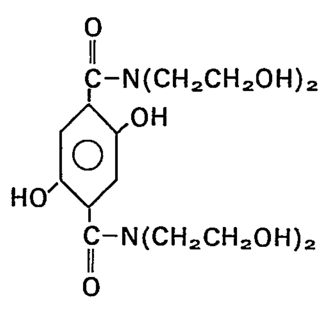

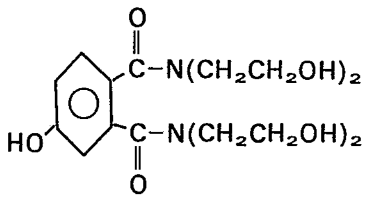

Innerhalb der Stoffklasse sind solche Hydroxyalkylderivate besonders wirksam, welche primäre Hydroxygruppen enthalten und ganz besonders solche, bei welchen die Hydroxyalkylgruppen Hydroxyethylgruppen darstellen. Diese Hydroxyethylamidderivate zeichnen sich durch eine besonders hohe zellöffnende Wirksamkeit bei bereits mässiger Dosierung (z.B. 0,5 bis 2,5 Gew.-%) aus.

Überraschenderweise ist die Wirksamkeit stark strukturabhängig. So sind z.B. Amide der Kohlensäure, z.B.

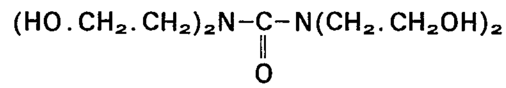

praktisch ohne zellöffnende Wirkung.

Zur Herstellung von Polyurethanschaumstoffen unter Mitverwendung der erfindungsgemäss zellöffnend wirksamen Polyhydroxyalkylamide mehrwertiger Carbonsäuren werden als schaumstoffbildende Ausgangsmaterialien eingesetzt.

1. Verbindungen mit mindestens zwei gegenüber Isocyanaten reaktionsfähigen Wasserstoffatomen von einem Molekulargewicht in der Regel von 400 bis 10 000. Hierunter versteht man neben Aminogruppen, Thiolgruppen oder Carboxylgruppen aufweisenden Verbindungen vorzugsweise Hydroxylgruppen aufweisende Verbindungen, insbesondere 2 bis 8 Hydroxylgruppen aufweisende Verbindungen, speziell solche vom Molekulargewicht 800 bis 6000, vorzugsweise 1500 bis 4000, vorzugsweise aber 2 bis 4 Hydroxylgruppen aufweisende Polyester, Polyether, Polythioether, Polyacetale, Polycarbonate und Polyesteramide, wie sie für die Herstellung von homogenen und zellförmigen Polyurethanen an sich bekannt sind und wie sie z.B. in der DE-A Nr. 2832253, S. 11 bis 18, beschrieben werden. Besonders bevorzugt sind Polyether, die durch Addition von einem oder mehreren Alkylenoxiden (Ethylenoxid und besonders Propylenoxid) an zwei- oder mehrwertige Starter), Propylenglykol, Glycerin, Sorbit, Formose, Triethanolamin, Trimethylolpropan erhalten werden, sowie Polyether, welche Polyadditionsprodukte aus Diisocyanaten und Hydrazin und/oder Diaminen und/oder Glykolen oder Polymerisate und/oder Pfropfpolymerisate, vorzugsweise aus Styrol und Acrylnitril dispergiert oder gelöst enthalten.

2. Gegebenenfalls werden als Ausgangsmaterialien Verbindungen mit mindestens 2 gegenüber Isocyanaten reaktionsfähigen Wasserstoffatomen und einem Molekulargewicht von 18 bis 399 mitverwendet. Auch in diesem Fall versteht man hierunter Hydroxylgruppen und/oder Aminogruppen und/oder Thiolgruppen und/oder Carboxylgruppen und/oder Hydrazidgruppen aufweisende Verbindungen, vorzugsweise Hydroxylgruppen und/oder Aminogruppen aufweisende Verbindungen, die als Kettenverlängerungsmittel oder Vernetzungsmittel dienen. Diese Verbindungen weisen in der Regel 2 bis 8, vorzugsweise 2 bis 4, gegenüber Isocyanaten reaktionsfähige Wasserstoffatome auf. Beispiele werden hierfür in der DE-A Nr. 2832253, S. 19-20, beschrieben. Genannt seien Wasser, Hydrazin, Butandiol-1,4, Trimethylolpropan, Formitgemische oder Adipinsäuredihydrazid.

3. Aliphatische, cycloaliphatische, araliphatische, heterocyclische und besonders aromatische Polyisocyanate, wie sie z.B. von W. Siefken in „Justus Liebigs Annalen der Chemie", 562, S. 75-136, beschrieben werden, beispielsweise solche der Formel $Q(NCO)_n$, in der n = 2 bis 4, vorzugsweise 2, und Q einen aliphatischen Kohlenwasserstoffrest mit 2 bis 18, vorzugsweise 6 bis 12 C-Atomen, einen cycloaliphatischen Kohlenwasserstoffrest mit 4 bis 20, vorzugsweise 5 bis 11 C-Atomen, einen aromatischen Kohlenwasserstoffrest mit 6 bis 20, vorzugsweise 6 bis 13 C-Atomen, oder einen araliphatischen Kohlenwasserstoffrest mit 8 bis 15, vorzugsweise 8 bis 13 C-Atomen bedeuten, z.B. solche Polyisocyanate wie sie in der DE-A Nr. 2832253, S. 10 bis 11 beschrieben werden. Besonders bevorzugt werden die technisch leicht zugänglichen Polyisocyanate, z.B. das 2,4- und/oder 2,6-Toluylendiisocyanat, sowie beliebige Gemische dieser Isomeren (TDI), Diphenylmethandiisocyanate (4,4'- und/oder 2,4'- und/oder 2,2'-Isomere), Polyphenylpolymethylenpolyisocyanate, wie sie durch Anilin/Formaldehyd-Kondensation und anschliessende Phosgenierung hergestellt werden (rohes MDI) und modifizierte Polyisocyanate, die z.B. Carbodiimidgruppen, Urethangruppen, Allophanatgruppen, Isocyanuratgruppen, Harnstoffgruppen und/oder Biuretgruppen aufweisen; insbesondere solche modifizierten Polyisocyanate, die sich vom 2,4- und/oder 2,6-Toluylendiisocyanat bzw. vom 4,4'- und/oder 2,4'-Diphenylmethandiisocyanat ableiten.

Gegebenfalls werden Hilfs- und Zusatzmittel wie leichtflüchtige anorganische oder organische Substanzen als Treibmittel, Katalysatoren der an sich bekannten Art, wie tertiäre Amine, Zinn(II)- und Zinn(IV)verbindungen, oberflächenaktive Zusatzstoffe, wie Emulgatoren und Schaumstabilisatoren, Reaktionsverzögerer, z.B. sauer reagierende Stoffe wie Salzsäure oder organische Säurehalogenide oder Säuren, ferner Zellregler der an sich bekannten Art wie Paraffine, Fettalkohole oder Dimethylpolysiloxane sowie Pigmente oder Farbstoffe und Flammschutzmittel der an sich bekannten Art, ferner Stabilisatoren gegen Alterungs-, Licht- und Witterungseinflüsse, Weichmacher und fungistatisch und bakteriostatisch wirkende Substanzen sowie Füllstoffe zugesetzt. Diese gegebenenfalls mitzuverwendenden Hilfs- und Zusatzstoffe werden beispielsweise in der DE-A Nr. 2732292, S. 21 bis 24, ausführlich beschrieben. Weitere Beispiele der Hilfs- und Zusatzmittel werden im Kunststoffhandbuch, B. VII, herausgegeben von Vieweg und Hoechteln, Carl-Hanser-Verlag, München 1966, S. 103 bis 113, beschrieben.

Die erfindungsgemäss einzusetzenden, zellöffnend wirkenden Polyhydroxyalkylamide werden üblicherweise mit der höhermolekularen Polyolverbindung, z.B. den Polyethern, in den angegebenen Mengen vermischt. Das Gemisch kann weiterhin die üblichen Zusatz- und Hilfsstoffe enthalten. Es ist jedoch auch möglich, die erfindungsgemässen zellöffnenden Substanzen zusammen mit Wasser und anderen Hilfsmitteln den Polyolen oder dem Reaktionsgemisch, das auch bereits vorgebildete NCO-Präpolymere darstellen kann, zuzumischen.

Die Menge an erfindungsgemäss zu verwendenden Zellöffnern liegt in dem bereits angegebenen Bereich, wird jedoch durch Reihenversuche innerhalb des Bereichs optimiert. Eine deutliche Überdosierung führt jedoch innerhalb der aufschäumenden Mischung zu einem Kollabieren des Schaums.

Die Schaumstoffherstellung erfolgt in üblicher Weise, technisch unter Zuhilfenahme von maschinellen Dosier- und Fördereinrichtungen. Hauptanwendungsgebiet ist die Herstellung von halbelastischen und elastischen Schaumstoffen nach dem Blockverschäumungsverfahren oder dem bekannten Doppeltransportbandverfahren.

Es ist jedoch gleichfalls möglich, statt des Freischaumes in Formen einzuschäumen und entsprechende Formschaumstoffe herzustellen. Dabei wird vorzugsweise in kalter Form geschäumt (kalthärtende Formschaumstoffe). Die Formverschäumung kann so durchgeführt werden, dass das Formteil an seiner Oberfläche Zellstruktur aufweist, sie kann aber auch so durchgeführt werden, dass das Formteil eine kompakte Haut und einen zelligen Kern aufweist. Dabei trägt man in die Form so viel schäumfähiges Reaktionsgemisch ein, dass der gebildete Schaumstoff die Form gerade ausfüllt, oder man dosiert mehr schäumfähiges Reaktionsgemisch in die Form, als zur Ausfällung des Forminneren notwendig ist. Die nach der Erfindung erhältlichen Produkte finden z.B. folgende Anwendung: Möbelpolsterungen, Matratzen, Automobilsitze, Armlehnen, Schwämme und Bauelemente sowie Sitz- und Armaturverkleidungen.

*Beispiele*

A. *Herstellung der Poly-(N,N-hydroxyalkyl) carbonamide*

*Beispiel 1:*

$$(HO-CH_2-CH_2)_2N-\overset{\overset{O}{\|}}{C}-\overset{\overset{O}{\|}}{C}-N(CH_2-CH_2-OH)_2$$

Zu 146 g (1 mol) Oxalsäurediethylester werden 210 g (2 mol) Diethanolamin getropft, wobei die Temperatur auf 90° C steigt und Ethanol fast quantitativ abdestilliert; man behandelt im Vakuum und erhält 264 g (quant.) an Tetrahydroxyethyloxalsäureamid; Visk. bei 80°C, 1100 cPo, OH-Zahl 850.

| *Analyse für* $C_{10}H_{20}N_2O_6$ (264): | | | |
|---|---|---|---|
| Berechnet: | C 45,4 | H 7,57 | N 10,6% |
| Gefunden: | C 45,0 | H 7,0 | N 10,4% |

*Beispiel 2:*

$$(HO-CH_2-CH_2)_2N-\overset{\overset{O}{\|}}{C}-CH_2-\overset{\overset{O}{\|}}{C}-N(CH_2-CH_2-OH)_2$$

Gemäss Beispiel 1 aus 160 g (1 mol) Malonsäurediethylester und 210 g (2 mol) Diethanolamin; Ausbeute 278 g (quant.) am Tetrahydroxyethylmalonsäureamid, OH-Zahl 800.

*Analyse für* $C_{11}H_{22}N_2O_6$ (278):

| | | | |
|---|---|---|---|
| Berechnet: | C 47,5 | H 7,9 | N 9,9% |
| Gefunden: | C 47,3 | H 7,5 | N 9,7% |

*Beispiel 3:*

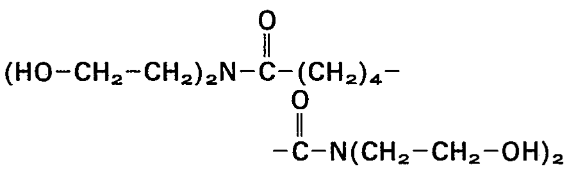

Gemäss Beispiel 1 aus 174 g (1 mol) Adipinsäuredimethylester und 210 g (2 mol) Diethanolamin; Ausbeute 320 g (quant.) eines zähen, langsam kristallisierenden Körpers, OH-Zahl 700.

*Analyse für* $C_{14}H_{28}N_2O_6$ (320):

| | | | |
|---|---|---|---|
| Berechnet: | C 52,5 | H 8,75 | N 8,75% |
| Gefunden: | C 52,0 | H 8,3 | N 8,4 % |

*Beispiel 4:*

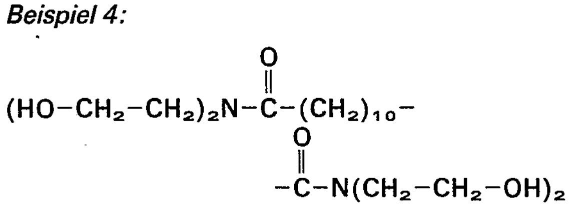

Gemäss Beispiel 1 aus 258 g (1 mol) Decandicarbonsäuredimethylester und 210 g (2 mol) Diethanolamin; Ausbeute 402 g, quantitativ einer hochviskosen Masse.

*Analyse für* $C_{20}H_{40}N_2O_6$ (404):

| | | | |
|---|---|---|---|
| Berechnet: | C 59,5 | H 9,88 | N 6,95% |
| Gefunden: | C 58,3 | H 9,30 | N 6,45% |

*Beispiel 5:*

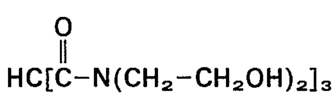

Gemäss Beispiel 1 aus 107 g (0,46 mol) Methantricarbonsäuretriethylester und 145 g (1,38 mol) Diethanolamin; Ausbeute 189 g (quant.), OH-Zahl 910.

*Analyse für* $C_{16}H_{31}N_3O_9$ (369):

| | | | |
|---|---|---|---|
| Berechnet: | C 52,0 | H 8,4 | N 11,4% |
| Gefunden: | C 51,5 | H 8,0 | N 11,2% |

*Beispiel 6:*

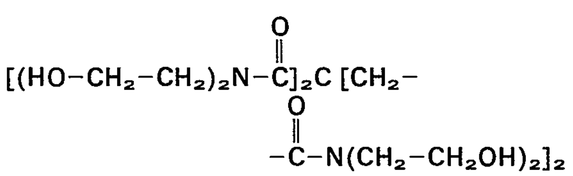

Gemäss Beispiel 1 aus 64,5 g (0,2 mol) 3,3-Bisethoxycarbonylglutarsäurediethylester und 84 g (0,8 mol) Diethanolamin; 106 g (quant.) hochviskoser Rückstand, OH-Zahl 800.

*Analyse für* $C_{22}H_{44}N_4O_{12}$ (556):

| | | | |
|---|---|---|---|
| Berechnet: | C 47,7 | H 7,9 | N 10,0% |
| Gefunden: | C 47,0 | H 7,4 | N 9,7% |

*Beispiel 7:*

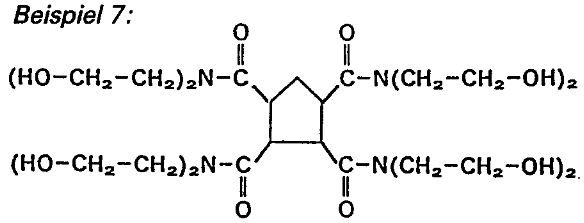

Gemäss Beispiel 1 aus 75,5 g (0,25 mol) 1,2,3,4-Cyclopentantetracarbonsäuremethylester und 105 g (1 mol) Diethanolamin; 146 g (quant.) Octahydroxyethyl-1,2,3,4-cyclopentantetracarbonsäureamid, OH-Zahl 750.

*Analyse für* $C_{25}H_{46}N_4O_{12}$ (593):

| | | | |
|---|---|---|---|
| Berechnet: | C 50,6 | H 7,6 | N 9,4% |
| Gefunden: | C 50,0 | H 7,1 | N 9,3% |

*Beispiel 8:*

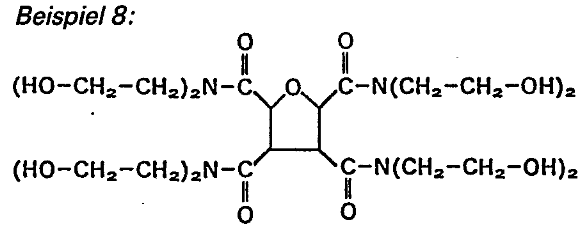

Gemäss Beispiel 1 aus 76 g (0,25 mol) Tetrahydrofuran-2,3,4,5-tetracarbonsäuremethylester und 105 g (1 mol) Diethanolamin; 149 g (quant.) Octahydroxyethyltetrahydrofuran-2,3,4,5-tetracarbonsäureamid, OH-Zahl 750.

*Analyse für* $C_{24}H_{44}N_4O_{13}$ (595):

| | | | |
|---|---|---|---|
| Berechnet: | C 48,4 | H 7,2 | N 9,4% |
| Gefunden: | C 48,2 | H 7,0 | N 9,2% |

*Beispiel 9:*

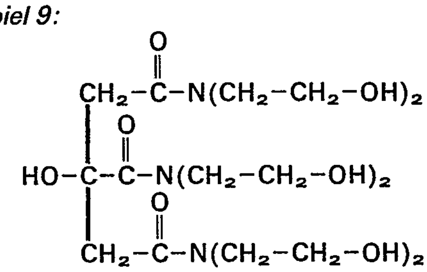

Gemäss Beispiel 1 aus 117 g (0,5 mol) Citronensäuretrimethylester und 157,5 g (1,5 mol) Diethanolamin; 220 g (quant.) Hexahydroxyethylcitronensäureamid, OH-Zahl 860.

*Analyse für* $C_{18}H_{35}N_3O_{10}$ (453):

| | | | |
|---|---|---|---|
| Berechnet: | C 47,6 | H 7,7 | N 9,3% |
| Gefunden: | C 47,1 | H 7,2 | N 9,1% |

*Beispiel 10:*

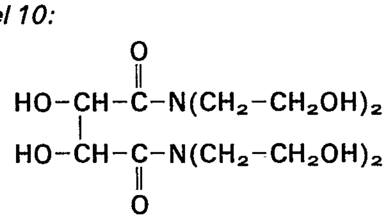

Gemäss Beispiel 1 aus 89 g (0,5 mol) Weinsäuredimethylester und 105 g (1 mol) Diethanolamin, Ausbeute 160 g (quant.) Tetrahydroxyethylweinsäureamid, OH-Zahl 1040.

*Analyse für* $C_{12}H_{24}N_2O_8$ (324):

| | | | |
|---|---|---|---|
| Berechnet: | C 44,4 | H 7,5 | N 8,6% |
| Gefunden: | C 44,0 | H 7,2 | N 8,3% |

*Beispiel 11:*

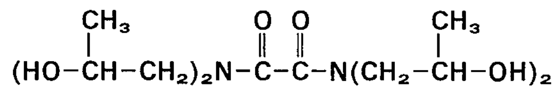

Gemäss Beispiel 1 aus 266 g (2 mol) Diisopropanolamin und 146 g (1 mol) Oxalsäurediethylester; Ausbeute 320 g (quant.) an Tetra-(2-hydroxypropyl)oxalsäureamid.

*Analyse für* $C_{14}H_{28}N_2O_6$ (320):

| | | | |
|---|---|---|---|
| Berechnet: | C 52,5 | H 8,75 | N 8,75% |
| Gefunden: | C 51,0 | H 8,4 | N 8,3 % |

*Beispiel 12:*

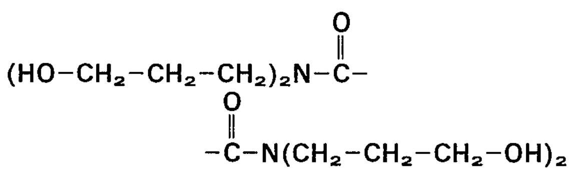

Gemäss Beispiel 1 aus 133 g (1 mol) Bis-(3-hydroxypropyl)amin und 73 g (0,5 mol) Oxalsäurediethylester; Ausbeute 160 g (quant.) an Tetra-(3-hydroxypropyl)oxalsäureamid.

*Analyse für* $C_{14}H_{28}N_2O_6$ (320):

| | | | |
|---|---|---|---|
| Berechnet: | C 52,5 | H 8,75 | N 8,75% |
| Gefunden: | C 51,3 | H 8,3 | N 8,6 % |

*Beispiel 12a:*

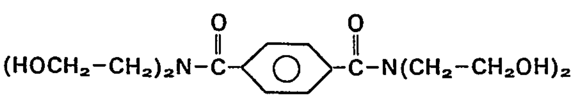

Aus 210 g (2 mol) Diethanolamin und 194 g (1 mol) Terephthalsäuredimethylester wird langsam Methanol abdestilliert, wobei eine klare Lösung entsteht; man behandelt zum Schluss bei 120 bis 140° C im Hochvakuum und erhält quantitativ das hochviskose Reaktionsprodukt, das nach längerem Stehen kristallin erstarrt. Es ist glatt löslich in Wasser und besitzt eine OH-Zahl von 656.

*Analyse für* $C_{16}H_{24}N_2O_6$ (340):

| | | | |
|---|---|---|---|
| Berechnet: | C 56,5 | H 7,06 | N 8,24% |
| Gefunden: | C 55,9 | H 6,90 | N 8,10% |

*Beispiel 12b:*

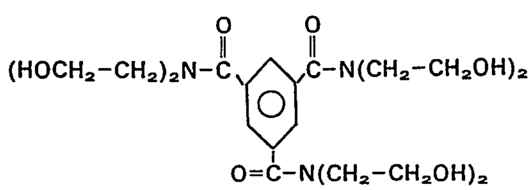

Aus 252 g (1 mol) Trimesinsäuretrimethylester und 315 g (3 mol) Diethanolamin wird langsam Methanol abdestilliert, wobei eine viskose, klare Lösung entsteht. Man legt anschliessend bei 120 bis 140° C Hochvakuum an, und erhält das glasartig erstarrende Reaktionsprodukt in quantitativer Ausbeute. Es ist glatt löslich in Wasser.

*Analyse für* $C_{21}H_{33}N_3O_9$ (471):

| | | | |
|---|---|---|---|
| Berechnet: | C 53,5 | H 7,0 | N 8,9% |
| Gefunden: | C 52,8 | H 6,8 | N 8,7% |

*Beispiel 12c:*

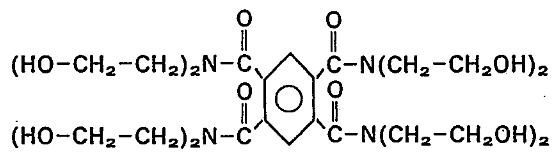

Aus 310 g (1 mol) Pyromellitsäuretetramethylester und 420 g (4 mol) Diethanolamin wird Methanol abdestilliert, wobei langsam eine klare, viskose Lösung entsteht. Zum Schluss wird bei 130 bis 140° C im Hochvakuum restliches Lösungsmittel abgezogen, bis man ein hochviskoses, glasartig erstarrendes Reaktionsprodukt von glatter Löslichkeit in Wasser und einer OH-Zahl von 745 erhält.

*Analyse für* $C_{26}H_{42}N_4O_{12}$ (602):

| | | | |
|---|---|---|---|
| Berechnet: | C 51,7 | H 6,97 | N 9,3% |
| Gefunden: | C 50,3 | H 6,80 | N 9,2% |

*Beispiel 12d:*

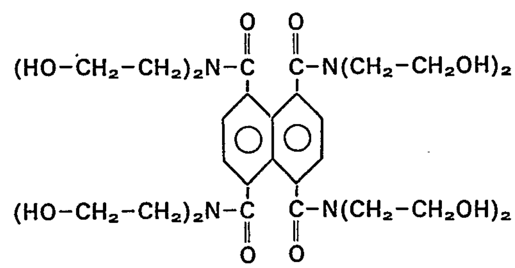

Aus 360 g (1 mol) 1,4,5,8-Naphthalintetracarbonsäuretetramethylester und 420 g (4 mol) Diethanolamin wird Methanol abdestilliert, wobei allmählich eine klare, viskose Lösung entsteht, aus der zum Schluss bei 140°C im Hochvakuum flüchtige Substanzen abgezogen werden. Man erhält quantitativ ein hochviskoses, glasartig erstarrendes Reaktionsprodukt von glatter Löslichkeit in Wasser und einer OH-Zahl von 685.

*Analyse für* $C_{30}H_{44}N_4O_{12}$ (652):

| | | | |
|---|---|---|---|
| Berechnet: | C 55,2 | H 6,75 | N 8,6% |
| Gefunden: | C 54,3 | H 6,55 | N 8,4% |

B. *Erfindungsgemässe Verwendung der Polyhydroxyalkylamide als Zellöffner in Polyurethanschaumrezepturen*

*Beispiel 13:*

Ein Gemisch bestehend aus

100 Gew.-Teilen eines Trimethylolpropangestarteten Poly(oxyethylenoxypropylen)triols des

mittleren Molgewichts 6000 und einer OH-Zahl von 28
3,0 Gew.-Teilen Wasser
0,2 Gew.-Teilen Triethylendiamin
3,6 Gew.-Teilen Diisopropanolamin
1,5 Gew.-Teilen Triethanolamin
2,0 Gew.-Teilen Trichlorethylphosphat
0,3 Gew.-Teilen eines chlorhaltigen Polysiloxanstabilisators
0,1 Gew.-Teilen Zinndioctoat
1,2 Gew.-Teilen Tetra(hydroxyethyl)oxalsäureamid als erfindungsgemäss eingesetzter Zellöffner (s. Beispiel 1)

wird mit 58,3 Gew.-Teilen eines allophanatisierten Toluylendiisocyanates des NCO-Gehaltes 40,5% auf einer kontinuierlichen Blockverschäumungsanlage vom Typ UBT zur Reaktion gebracht. Es entsteht ein offenzelliger, nichtschrumpfender, hochelastischer Schaumstoff.

*Vergleichsbeispiel 13a:*

Zum Vergleich wurde der in Beispiel 13 genannte Schaumstoff ohne Zusatz von Tetrahydroxyethyloxalsäureamid hergestellt. Der daraus resultierende Schaumstoff war geschlossenzellig und zeigt beim Abkühlen starke Schrumpferscheinung.

*Vergleichsbeispiel 13b:*

Zum weiteren Vergleich wurde der in Beispiel 13 genannte Schaumstoff ohne Zusatz von Tetrahydroxyethyloxalsäureamid und ohne Zusatz von Zinndioctoat hergestellt. Der so hergestellte hochelastische Schaumstoff zeigte nur geringe Oberflächenschrumpferscheinungen. Die Zug- und Bruchdehnungseigenschaften sind jedoch deutlich schlechter als in Beispiel 13.

In Tabelle 1 sind die mechanischen Daten der aus Beispiel 13 und Vergleichsbeispiel 13b resultierenden Schaumstoffe gegenübergestellt:

*Tabelle 1*

| | Beispiel 13 | Vergleich 13b |
|---|---|---|
| Rohdichte (kg/m³) | 35 | 34 |
| Zugfestigkeit (kPa) | 115 | 90 |
| Bruchdehnung (%) | 140 | 110 |
| Stauchhärte (kPa) bei 40% Kompr. | 2,4 | 2,4 |

*Vergleichsbeispiel 13c:*

Verwendet man ein Gemisch wie in Beispiel 13, jedoch mit 11 Gew.-Teilen Tetrahydroxyethyloxalsäureamid, so kollabiert das schäumfähige Gemisch während des Aufsteigvorgangs.

*Beispiel 14:*

Ein Gemisch, bestehend aus
100 Gew.-Teilen eines trimethylolpropangestarteten Poly(oxyethylenoxypropylen)-triols der OH-Zahl 35
2,6 Gew.-Teilen Wasser
0,133 Gew.-Teilen Triethylendiamin
0,2 Gew.-Teilen Dimethylaminoethylether
0,2 Gew.-Teilen eines chlorhaltigen Polysiloxanstabilisators
2,0 Gew.-Teilen einer 80%igen Lösung von Octahydroxyethyl-1,2,3,4-cyclopentantetracarbonsäureamid in Wasser (s. Beispiel 7)

wird mit 53,5 Gew.-Teilen eines Diphenylmethandiisocyanatgemisches mit einem Zweikerngehalt von 85% und einem NCO-Gehalt von 32,5% auf einer kontinuierlichen Blockverschäumungsanlage vom Typ UBT zur Reaktion gebracht. Es entsteht ein offenzelliger, nichtschrumpfender, elastischer Schaumstoff mit folgenden mechanischen Eigenschaften:

| | |
|---|---|
| Rohdichte (kg/m³) | 35 |
| Zugfestigkeit (kPa) | 105 |
| Bruchdehnung (%) | 130 |
| Stauchhärte (kPa) bei 40% Kompr. | 2,7 |

*Vergleichsbeispiel 14a:*

Zum Vergleich wurde wie in Beispiel 14 ein Schaumstoff ohne Zusatz von Octahydroxyethyl-1,2,3,4-cyclopentantetracarbonsäureamid hergestellt. Der dabei erhaltene Schaumstoff zeigte nach Abkühlen Totalschrumpf.

*Beispiel 15:*

Ein Gemisch, bestehend aus
66 Gew.-Teilen eines trimethylolpropangestarteten Poly(oxyethylenoxypropylen)-triols mit der OH-Zahl 35
33 Gew.-Teilen eines trimethylolpropangestarteten Poly(oxyethylenoxypropylen)-triols, welches 25% eines polymeren Füllstoffes aus Acrylnitril/Styrol enthält und eine OH-Zahl von 26 besitzt
3,0 Gew.-Teilen Wasser
0,15 Gew.-Teilen einer 33%igen Lösung von Triethylendiamin in Dipropylenglykol
0,1 Gew.-Teilen Dimethylaminoethylether
1,5 Gew.-Teilen Diethanolamin
0,5 Gew.-Teilen eines chlorhaltigen Polysiloxanstabilisators
0,1 Gew.-Teilen Zinndioctoat
1,0 Gew.-Teilen Tetrahydroxyethylmalonsäureamid (s. Beispiel 2)

wird mit 38,7 Gew.-Teilen Toluylendiisocyanat 80/20 in einem offenen Gefäss zur Reaktion gebracht. Es entsteht ein offenzelliger, nichtschrumpfender, hochelastischer Schaumkörper mit folgenden mechanischen Eigenschaften:

| | |
|---|---|
| Rohdichte (kg/m³) | 32 |
| Zugfestigkeit (kPa) | 110 |
| Bruchdehnung (%) | 164 |
| Stauchhärte (kPa) bei 40% Kompr. | 2,5 |

Ein Vergleichsschaum ohne Tetrahydroxyethylmalonsäureamid ist stark geschlossenzellig und muss vor dem Erkalten mechanisch aufgedrückt werden.

*Beispiel 16:*

Ein Gemisch, bestehend aus

- 100 Gew.-Teilen eines trimethylolpropangestarteten Poly(oxyethylenoxypropylen)-triols des mittleren Molgewichtes 6000 und einer OH-Zahl von 28
- 3,0 Gew.-Teilen Wasser
- 0,2 Gew.-Teilen Triethylendiamin
- 3,6 Gew.-Teilen Diisopropanolamin
- 1,5 Gew.-Teilen Triethanolamin
- 2,0 Gew.-Teilen Trichlorethylphosphat
- 0,3 Gew.-Teilen eines chlorhaltigen Polysiloxanstabilisators
- 0,1 Gew.-Teilen Zinndioctoat
- 1,2 Gew.-Teilen Tetra-(3-hydroxypropyl)-oxalsäureamid als erfindungsgemäss eingesetzter Zellöffner (s. Beispiel 12)

wird mit 58,3 Gew.-Teilen eines allophanatisierten Toluylendiisocyanates des NCO-Gehaltes 40,5% auf einer kontinuierlichen Blockverschäumungsanlage vom Typ UBT zur Reaktion gebracht. Es entsteht ein offenzelliger, nichtschrumpfender, hochelastischer Schaumstoff.

*Beispiel 17:*

Ein Gemisch aus

- 100 Gew.-Teilen eines trimethylolpropangestarteten Poly(oxyethylenoxypropylen)-triols des mittleren Molekulargewichts 6000 und einer OH-Zahl von 28
- 3,0 Gew.-Teilen Wasser
- 0,2 Gew.-Teilen Triethylendiamin
- 3,6 Gew.-Teilen Diisopropanolamin
- 1,5 Gew.-Teilen Triethanolamin
- 2,0 Gew.-Teilen Trichlorethylphosphat
- 0,3 Gew.-Teilen eines chlorhaltigen Polysiloxanstabilisators
- 0,1 Gew.-Teilen Zinndioctoat
- 1,5 Gew.-Teilen einer 80%igen Lösung von Tetra(hydroxyethyl)terephthalsäureamid in Wasser (s. Beispiel 12a) als erfindungsgemäss eingesetzter Zellöffner

wird mit 58,3 Gew.-Teilen eines allophanatisierten Toluylendiisocyanates des NCO-Gehaltes 40,5% intensiv vermischt und in einer offenen Form verschäumt. Es entsteht ein offenzelliger, nichtschrumpfender, hochelastischer Schaumstoff.

*Beispiel 18:*

Ein Gemisch aus

- 100 Gew.-Teilen eines trimethylolpropangestarteten Poly(oxyethylenoxypropylen)-triols des mittleren Molekulargewichts 6000 und einer OH-Zahl von 28
- 3,0 Gew.-Teilen Wasser
- 0,2 Gew.-Teilen Triethylendiamin
- 3,6 Gew.-Teilen Diisopropanolamin
- 1,5 Gew.-Teilen Triethanolamin
- 2,0 Gew.-Teilen Trichlorethylphosphat
- 0,3 Gew.-Teilen eines chlorhaltigen Polysiloxanstabilisators
- 0,1 Gew.-Teilen Zinndioctoat
- 1,5 Gew.-Teilen einer 80%igen Lösung von Hexa(hydroxyethyl)trimesinsäureamid in Wasser (s. Beispiel 12b) als erfindungsgemäss eingesetzter Zellöffner

wird mit 58,3 Gew.-Teilen eines allophanatisierten Toluylendiisocyanates des NCO-Gehaltes 40,5% intensiv vermischt und in einer offenen Form verschäumt. Es entsteht ein offenzelliger, nichtschrumpfender, hochelastischer Schaumstoff.

*Beispiel 19:*

Ein Gemisch aus

- 100 Gew.-Teilen des Polyethers von Beispiel 17
- 3,0 Gew.-Teilen Wasser
- 0,2 Gew.-Teilen Triethylendiamin
- 3,6 Gew.-Teilen Diisopropanolamin
- 1,5 Gew.-Teilen Triethanolamin
- 2,0 Gew.-Teilen Trichlorethylphosphat
- 0,3 Gew.-Teilen eines chlorhaltigen Polysiloxanstabilisators
- 0,1 Gew.-Teilen Zinndioctoat
- 1,5 Gew.-Teilen einer 80%igen Lösung von Octa(hydroxyethyl)pyromellithsäureamid in Wasser (s. Beispiel 12c) als erfindungsgemäss eingesetzter Zellöffner

wird mit 58,3 Gew.-Teilen eines allophanatisierten Toluylendiisocyanates des NCO-Gehaltes 40,5% intensiv vermischt und in einer offenen Form verschäumt. Es entsteht ein offenzelliger, nichtschrumpfender, hochelastischer Schaumstoff.

**Patentansprüche**

1. Verwendung von Poly-N,N-hydroxyalkyl amiden mehrwertiger Polycarbonsäuren bei der Herstellung von Polyurethanschaumstoffen, dadurch gekennzeichnet, dass man für die Herstellung von offenzelligen, elastischen Polyurethanschaumstoffen durch Umsetzung von mindestens 2 gegenüber Isocyanaten reaktionsfähige Wasserstoffatome aufweisenden Verbindungen vom Molekulargewicht 400 bis 10 000 mit Polyisocyanaten und gegebenenfalls Kettenverlängerungsmitteln vom Molekulargewicht 18 bis 400 in Gegenwart von Katalysatoren und Wasser als Treibmittel und gegebenenfalls an sich bekannten Hilfs- und Zusatzmitteln, Poly-N,N-hydroxyalkylamide mehrwertiger aliphatischer, cycloaliphatischer und/oder aromatischer Polycarbonsäuren der Formel

$$X\left[-\underset{\underset{O}{\parallel}}{C}-N\left(A-OH\right)_2\right]_n$$

wobei n eine ganze Zahl von 2 bis 6, X nur eine Bindung (bei n = 2), ein n-wertiger $C_1$ bis $C_{10}$-geradkettiger oder verzweigter, gegebenenfalls hydroxylgruppensubstituierter Alkanrest, ein $C_4$ bis $C_6$-Cycloalkanrest, der im Ring gegebenenfalls O, S oder $N-CH_3$ als Heteroatom oder Heteroatomgruppe enthält, ein n-wertiger $C_6$ bis $C_{20}$-Arylrest, der gegebenenfalls durch eine oder mehrere Hydroxylgruppen substituiert ist und A eine geradkettige oder verzweigte, gegebenenfalls eine OH-Gruppe aufweisende $C_2$ bis $C_6$-Alkylengruppe, darstellt,

in Mengen von 0,1 bis 7,5, bevorzugt 0,5 bis 5 Gew.-%, bezogen auf das Gemisch mit den mindestens 2 gegenüber Isocyanaten reaktionsfähige Wasserstoffe aufweisenden Verbindungen vom Molekulargewicht 400 bis 10 000, als zellöffnende Komponente einsetzt.

2. Verwendung nach Anspruch 1, dadurch gekennzeichnet, dass man als Polyisocyanate modifizierte, verzweigte, aromatische Diisocyanattypen mit Allophanat-, Biuret-, Isocyanurat-, Carbodiimid-, Urethan- und/oder Harnstoffgruppen verwendet und solche Poly-N,N-hydroxyalkylamide mehrwertiger Carbonsäuren verwendet, bei welchen n und X die in Anspruch 1 angegebene Bedeutung haben und A den $-CH_2-CH_2-$-und/oder $CH_2-CH_2-CH_2-$-Rest bedeutet und diese zusammen mit metallorganischen Katalysatoren einsetzt.

3. Verwendung nach den Ansprüchen 1 und 2, dadurch gekennzeichnet, dass man als Diisocyanate modifizierte Polyisocyanate, welche Carbodiimidgruppen, Urethangruppen, Allophanatgruppen, Isocyanuratgruppen, Harnstoffgruppen und/oder Biuretgruppen aufweisen und sich vom 2,4- und/oder 2,6-Toluylendiisocyanat bzw. vom 4,4'- und/oder 2,4'-Diphenylmethandiisocyanat ableiten, verwendet.

4. Verwendung nach den Ansprüchen 1 bis 3, dadurch gekennzeichnet, dass man Poly-N,N-hydroxyalkylamide mehrwertiger Carbonsäuren, wobei n und X die in den Ansprüchen 1 und 2 angegebene Bedeutung haben und A den $-CH_2-CH_2-$-Rest bedeutet, in Mengen von 0,5 bis 2,5 Gew.-%, bezogen auf das Gemisch mit mindestens 2 gegenüber Isocyanaten reaktionsfähige Wasserstoffe aufweisenden Verbindungen vom Molekulargewicht 400 bis 10 000, als zellöffnende Komponente einsetzt.

5. Verwendung nach den Ansprüchen 1 bis 4, dadurch gekennzeichnet, dass man als Zellöffner das Tetrahydroxyethylmalonsäureamid verwendet.

6. Verwendung nach den Ansprüchen 1 bis 5, dadurch gekennzeichnet, dass man die Umsetzung mit Organozinnverbindungen katalysiert.

## Claims

1. Use of poly-N,N-hydroxyalkylamides of polybasic polycarboxylic acids in the production of polyurethane foams, characterised in that for the production of open-cell, elastic polyurethane foams by the reaction of compounds containing at least two isocyanate-reactive hydrogen atoms and having a molecular weight of 400 to 10,000 with polyisocyanates and, optionally, chain-extending agents having a molecular weight of 18 to 400 in the presence of catalysts and water as the blowing agent and optionally auxiliaries and additives known *per se*, poly-N,N-hydroxyalkylamides of polybasic aliphatic, cycloaliphatic and/or aromatic polycarboxylic acids of the formula

$$X\left[-\underset{\underset{O}{\|}}{C}-N(A-OH)_2\right]_n$$

wherein n represents an integer from 2 to 6, X represents only one bond (where n = 2), an n-functional $C_1$ to $C_{10}$ straight-chain or branched alkane radical optionally substituted by hydroxyl groups, a $C_4$ to $C_6$-cycloalkane radical which optionally contains O, S or $N-CH_3$ as hetero atom or group of hetero atoms in the ring, or an n-functional $C_6$ to $C_{20}$-aryl radical which is optionally substituted by one or more hydroxyl groups, and A represents a straight-chain or branched $C_2$ to $C_6$-alkylene group optionally containing an OH group,
are used as the cell-opening component in quantities of 0.1 to 7.5% by weight, preferably 0.5 to 5% by weight, based on the mixture with the compounds containing at least two isocyanate-reactive hydrogen atoms and having a molecular weight of 400 to 10,000.

2. Use according to Claim 1, characterised in that the polyisocyanates used are modified, branched aromatic types of diisocyanate containing allophanate, biuret, isocyanurate, carbodiimide, urethane and/or urea groups and the poly-N,N-hydroxyalkylamides of polybasic carboxylic acids used are those in which n and X have the meaning indicated in Claim 1 and A denotes the $-CH_2-CH_2-$- and/or $CH_2-CH_2-CH_2$-radical and these are used together with organometallic catalysts.

3. Use according to Claims 1 and 2, characterised in that the diisocyanates used are modified polyisocyanates which contain carbodiimide groups, urethane groups, allophanate groups, isocyanurate groups, urea groups and/or biuret groups and are derived from 2,4- and/or 2,6-tolylene diisocyanate or from 4,4'- and/or 2,4'-diphenylmethane diisocyanate.

4. Use according to Claims 1 to 3, characterised in that poly-N,N-hydroxyalkylamides of polybasic carboxylic acids, wherein n and X have the meaning indicated in Claims 1 and 2 and A denotes the $-CH_2-CH_2-$-radical, are used as the cell-opening component in quantities of 0.5 to 2.5% by weight, based on the mixture with compounds containing at least 2 isocyanate-reactive hydrogen atoms and having a molecular weight of 400 to 10,000.

5. Use according to Claims 1 to 4, characterised in that tetrahydroxyethyl malonic acid amide is used as the cell-opener.

6. Use according to Claims 1 to 5, characterised in that the reaction is catalysed with organotin compounds.

## Revendications

1. Utilisation de poly-N,N-hydroxyalkylamides d'acides polycarboxyliques polyvalents lors de la fabrication de mousses de polyuréthannes, caractérisée en ce que, pour la fabrication de mousses

de polyuréthannes élastiques à cellules ouvertes par réaction de composés comportant au moins deux atomes d'hydrogène réactifs vis-à-vis des isocyanates et ayant un poids moléculaire de 400 à 10 000 avec des polyisocyanates et éventuellement des agents d'allongement de chaîne d'un poids moléculaire de 18 à 400, en présence de catalyseurs et d'eau comme agent moussant et éventuellement en présence de substances auxiliaires et d'additifs connus en soi, on utilise des poly-N,N-hydroxyalkylamides d'acides polycarboxyliques aromatiques, cycloaliphatiques et/ou aliphatiques polyvalents de formule

$$X\left[\begin{array}{l}-C-N\left(A-OH\right)_2\\ \quad\|\\ \quad O\end{array}\right]_n$$

dans laquelle n représente un nombre entier de 2 à 6, X représente une seule liaison (lorsque n = 2), un groupe alcane n-valent en $C_1$ à $C_{10}$, à chaîne droite ou ramifiée et éventuellement substitué par des groupes hydroxy, un groupe cycloalcane en $C_4$ à $C_6$ contenant éventuellement, dans le noyau, O, S ou $N-CH_3$ comme hétéroatome ou groupe d'hétéroatomes, un groupe aryle n-valent en $C_6$ à $C_{20}$ qui est éventuellement substitué par un ou plusieurs groupes hydroxy et A représente un groupe alkylène en $C_2$ à $C_6$ à chaîne droite ou ramifiée et comportant éventuellement un groupe OH,
en quantités de 0,1 à 7,5% en poids, de préférence, de 0,5 à 5% en poids, calculé sur le mélange avec les composés comportant au moins deux atomes d'hydrogène réactifs vis-à-vis des isocyanates et ayant un poids moléculaire de 400 à 10 000 comme composant ouvrant les cellules.

2. Utilisation suivant la revendication 1, caractérisée en ce que, comme polyisocyanates, on utilise des types de diisocyanates aromatiques modifiés et ramifiés comportant des groupes allophanate, biuret, isocyanurate, carbodiimide, uréthanne et/ou urée, tandis que l'on utilise des poly-N,N-hydroxyalkylamides d'acides carboxyliques polyvalents, dans lesquels n et X ont les significations indiquées dans la revendication 1 et A représente le radical $-CH_2-CH_2-$ et/ou le radical $CH_2-CH_2-CH_2$, et ce conjointement avec des catalyseurs organométalliques.

3. Utilisation suivant les revendications 1 et 2, caractérisée en ce que, comme diisocyanates, on utilise des polyisocyanates modifiés comportant des groupes carbodiimide, des groupes uréthanne, des groupes allophanate, des groupes isocyanurate, des groupes urée et/ou des groupes biuret et dérivant du 2,4- et/ou du 2,6-toluylènediisocyanate ou du 4,4'- et/ou du 2,4'-diphénylméthane-diisocyanate.

4. Utilisation suivant les revendications 1 à 3, caractérisée en ce que, comme composant ouvrant les cellules, on utilise des poly-N,N-hydroxyalkylamides d'acides carboxyliques polyvalents, n et X ayant les significations indiquées dans les revendications 1 et 2 et A représentant le radical $-CH_2-CH_2-$, en quantités de 0,5 à 2,5% en poids, calculé sur le mélange avec des composés comportant au moins deux atomes d'hydrogène réactifs vis-à-vis des isocyanates et ayant un poids moléculaire de 400 à 10 000.

5. Utilisation suivant les revendications 1 à 4, caractérisée en ce que, comme agent d'ouverture de cellules, on utilise l'amide d'acide tétrahydroxyéthylmalonique.

6. Utilisation suivant les revendications 1 à 5, caractérisée en ce qu'on catalyse la réaction avec des composés organiques d'étain.